Europäisches Patentamt

European Patent Office

Office européen des brevets

(19)

(11) Numéro de publication : **0 337 838 B1**

# (12) FASCICULE DE BREVET EUROPEEN

(45) Date de publication du fascicule du brevet :
**11.12.91 Bulletin 91/50**

(51) Int. Cl.⁵ : **C07C 321/16**

(21) Numéro de dépôt : **89400843.2**

(22) Date de dépôt : **24.03.89**

(54) **Synthèse du benzylmercaptan.**

(30) Priorité : **14.04.88 FR 8804963**

(43) Date de publication de la demande :
**18.10.89 Bulletin 89/42**

(45) Mention de la délivrance du brevet :
**11.12.91 Bulletin 91/50**

(84) Etats contractants désignés :
**AT BE CH DE ES GB GR IT LI LU NL SE**

(56) Documents cités :
**US-A- 4 082 790**

(73) Titulaire : **SOCIETE NATIONALE ELF
AQUITAINE (PRODUCTION)
Tour Elf, 2, Place de la Coupole, La Défense 6
F-92400 Courbevoie (FR)**

(72) Inventeur : **Labat, Yves
35 bis, Rue Michel Hourau
F-64000 Pau (FR)**

(74) Mandataire : **Leboulenger, Jean et al
ATOCHEM Département Propriété Industrielle
F-92091 Paris la Défense 10 Cédex 42 (FR)**

## Description

La présente invention concerne le domaine des mercaptans et a plus particulièrement pour objet la préparation du benzylmercaptan également connu sous le nom d'alcool thiobenzylique.

Le benzylmercaptan est notamment utile comme matière première pour la synthèse d'herbicides de la famille des thiocarbamates. Pour cette application, une excellente pureté du benzylmercaptan est exigée.

La synthèse du benzylmercaptan par action de l'hydrogène sulfuré sur l'alcool benzylique en présence d'un catalyseur n'est pas économiquement viable en raison du prix élevé de l'alcool benzylique et de son mauvais comportement en catalyse (désactivation des catalyseurs et perte rapide de sélectivité).

Une voie de synthèse économiquement plus attrayante est la substitution nucléophile de l'atome de chlore du chlorure de benzyle par un sulfhydrate alcalin, le sulfhydrate d'ammonium ou un sulfhydrate d'amine. Cette réaction, généralement effectuée en milieu alcoolique, est connue depuis longtemps :

— C. Märcker, Liebigs Ann. Chem. 136, 76 (1865)

— Brevet US 2 456 588 (1948)

— H. Zinner, Chem. Ber. 86, 825 (1953)

— J.E. Bittell et al., J. Org. Chem. 43, 1687 (1978)

— Brevet US 4 082 790 (1978) mais les rendements obtenus sont moyens et la conversion n'est pas complète. Selon le brevet US 4 082 790 précité, l'utilisation du sulfhydrate de butylamine en milieu isopropanolique donne du mélange contenant 10% de chlorure de benzyle non transformé, 82% de benzylmercaptan et 6% de sulfure de benzyle, et l'emploi du sulfhydrate d'ammonium en milieu méthanolique conduit à un mélange contenant 92% de benzylmercaptan et 8% de sulfure de benzyle.

Les procédés antérieurs ne permettent pas l'obtention économique d'un benzylmercaptan satisfaisant aux spécifications requises par les utilisateurs, notamment pureté supérieure à 98% et teneur en chlorure de benzyle inférieure à 0,1%. En effet, la séparation du chlorure de benzyle par distillation du benzylmercaptan brut est très difficile. D'autre part, la formation de sous-produits (sulfure de benzyle et accessoirement disulfure de benzyle), bien que séparables par distillation, est préjudiciable à l'économie du procédé. Enfin, l'emploi d'un solvant alcoolique qu'il faut recycler ou détruire avant rejet, complique l'installation de production.

Il existe donc un besoin pour un procédé de fabrication du benzylmercaptan permettant à la fois une conversion quasi-totale du chlorure de benzyle et une bonne sélectivité en benzylmercaptan, et ceci sans utilisation d'alcool comme milieu réactionnel.

Il a maintenant été trouvé qu'on peut parvenir à ce résultat avec le sulfhydrate d'ammonium à condition d'opérer dans des conditions spécifiques, notamment sous pression autogène en réacteur fermé et avec un profil de température adapté.

Plus précisément, l'invention a pour objet un procédé de préparation du benzylmercaptan par réaction du chlorure de benzyle et du sulfhydrate d'ammonium dans un rapport molaire $NH_4SH/C_6H_5CH_2Cl$ au moins égal à 1, de préférence compris entre environ 1,05 et 1,5, caractérisé en ce que la réaction est effectuée sous pression autogène en réacteur fermé en deux étapes, la première consistant à introduire la chlorure de benzyle dans une solution aqueuse de sulfhydrate d'ammonium à une température inférieure à 80°C et la seconde à chauffer le mélange réactionnel à une température allant de 80 à 100°C.

La concentration de la solution aqueuse de sulfhydrate d'ammonium peut varier dans de larges limites car elle n'a pas d'effet sensible sur le rendement ou la conversion. Cependant, dans la pratique, il est avantageux d'opérer à une concentration en $NH_4SH$ d'au moins 25% en poids pour assurer un relargage à chaud de la phase organique et par conséquent une décantation satisfaisante. On utilise donc de préférence une solution aqueuse ayant une concentration en $NH_4SH$ comprise entre 25 et 40%.

L'équation réactionnelle étant :
$$NH_4SH + C_6H_5CH_2Cl \rightarrow C_6H_5CH_2\text{-}SH + NH_4Cl$$

il est évident que le rapport molaire $NH_4SH/C_6H_5CH_2Cl$ doit au moins être égal à 1. Pour favoriser la conversion complète du chlorure de benzyle, il est souhaitable d'employer au moins un léger excès de sulfhydrate d'ammonium (par exemple, environ 1,05). Un rapport molaire $NH_4SH/C_6H_5CH_2Cl$ supérieur à 1,5 est possible, mais n'apporte aucune amélioration ; il conduit par contre inutilement à une pression plus élevée due à la décomposition du $NH_4SH$ :

$$NH_4SH \rightarrow H_2S + NH_3$$

Le rapport molaire optimal se situe aux alentours de 1,2.

Conformément au procédé selon l'invention, l'introduction du chlorure de benzyle dans la solution aqueuse de sulfhydrate d'ammonium doit être effectuée à une température inférieure à 80°C, notamment entre 0 et 80°C et de préférence à la température ambiante. Durant l'introduction, on peut soit maintenir la température à la valeur choisie, soit la faire évoluer pour atteindre 80°C en fin d'introduction en mettant à profit l'exothermicité de la réaction. La durée d'introduction de chlorure de benzyle ne semble pas avoir d'influence sur les résultats ; elle est généralement comprise entre environ 15 minutes et une heure.

Une fois l'introduction terminée, le mélange réactionnel est maintenu à une température allant de 80 à 100°C, de préférence à 80-90°C, jusqu'à la conversion quasi-complète du chlorure de benzyle. La durée de cette opération est généralement comprise entre 1 et 3 heures, la durée optimale étant d'environ 2 heures.

Après soutirage de la phase aqueuse, la phase organique est avantageusement strippée à l'azote pour éliminer l'hydrogène sulfuré résiduel. Le produit brut ainsi obtenu contient généralement plus de 95% de benzylmercaptan et moins de 0,1% de chlorure de benzyle résiduel. Par distillation de ce brut sous pression réduite, on obtient finalement un benzylmercaptan de pureté supérieure à 99%.

Les exemples suivants illustrent l'invention sans la limiter.

## EXEMPLE 1

Dans un réacteur thermostaté de 50 litres en acier inoxydable, on charge 17240 g de solution ammoniacale à 11,8% (soit 120 moles de $NH_3$), puis on introduit de l'hydrogène sulfuré (120 moles) jusqu'à absorption totale correspondant à la formation de $NH_4SH$. La température de la solution de $NH_4SH$ étant maintenue à environ 16°C et le réacteur fermé, on introduit en 30 minutes 12766 g de chlorure de benzyle à 99% de pureté (soit 100 moles). La pression monte à 3 bars. On porte ensuite la température à 80°C et on l'y maintient pendant 2 heures. La pression monte à 7 bars, puis redescend à 5 bars.

Après soutirage de la phase aqueuse à chaud, on refroidit la phase organique et on effectue un strippage à l'azote pour éliminer l'hydrogène sulfuré résiduel. On obtient ainsi 12405 g d'un produit brut dont l'analyse chromatographie montre qu'il contient 96,7% de benzylmercaptan, 0,02% de chlorure de benzyle, 1% de sulfure de benzyle et 1,7% de disulfure de benzyle, soit un rendement de 96,7% par rapport au chlorure de benzyle mis en oeuvre.

Par distillation de ce produit brut sous 2666 Pa, on obtient un benzylmercaptan de pureté supérieure à 99%.

## EXEMPLE 2

On opère comme à l'exemple 1 sauf que l'introduction de chlorure de benzyle est effectuée à 60°C. Durant le chauffage à 80°C, la pression monte à 6 bars, puis redescend à 4 bars.

Le produit brut ainsi obtenu contient 97% de benzylmercaptan, moins de 0,1% de chlorure de benzyle, 1% de sulfure de benzyle et 1,5% de disulfure de benzyle.

## EXEMPLE 3

En opérant comme à l'exemple 1, mais avec seulement 104 moles de $NH_4SH$, on obtient un produit brut contenant 96,3% de benzylmercaptan, moins de 0,1% de chlorure de benzyle, 1,3% de sulfure de benzyle et 1,5% de disulfure de benzyle.

## EXEMPLE 4

On répète l'exemple 1 dans les conditions suivantes :
— $NH_4SH$ : 150 moles
— introduction du chlorure de benzyle (100 moles) à 20°C
— chauffage à 90°C pendant 2 heures

La pression monte à 12 bars, puis redescend à 9 bars. Le produit brut obtenu contient 96% de benzylmercaptan, moins de 0,1% de chlorure de benzyle, 0,8% de sulfure de benzyle et 2,1% de disulfure de benzyle.

## Revendications

1. Procédé de préparation du benzylmercaptan par réaction du chlorure de benzyle avec le sulfhydrate d'ammonium dans un rapport molaire $NH_4SH/C_6H_5CH_2Cl$ au moins égal à 1, caractérisé en ce que l'on effectue la réaction sous pression autogène en réacteur fermé en deux étapes, la première consistant à introduire le chlorure de benzyle dans une solution aqueuse de sulfhydrate d'ammonium à une température inférieure à 80°C et la seconde à chauffer le mélange réactionnel à une température allant de 80 à 100°C.

2. Procédé selon la revendication 1, dans lequel la concentration de la solution aqueuse de sulfhydrate d'ammonium est comprise entre 25 et 40% en poids.

3. Procédé selon la revendication 1 ou 2, dans lequel le rapport molaire $NH_4SH/C_6H_5CH_2Cl$ est compris entre environ 1,05 et 1,5, et de préférence voisin de 1,2.

4. Procédé selon l'une des revendications 1 à 3, dans lequel durant l'introduction de chlorure de benzyle on laisse évoluer la température de façon à atteindre 80°C en fin d'introduction.

## Claims

1. Process for the preparation of benzyl mercaptan by reaction of benzyl chloride with ammonium hydrosulphide in an $NH_4SH/C_6H_5CH_2Cl$ molar ratio of at least 1, characterised in that the reaction is carried out at autogenous pressure in a closed reactor in two stages, the first consisting in introducing benzyl chloride into an aqueous solution of ammonium hydrosulphide at a temperature below 80°C, and the second in heating the reaction mixture to a temperature ranging from 80 to 100°C.

2. Process according to Claim 1, in which the concentration of the aqueous solution of ammonium hydrosulphide is between 25 and 40% by weight.

3. Process according to Claim 1 or 2, in which the $NH_4SH/C_6H_5CH_2Cl$ molar ratio is between approximately 1.05 and 1.5, and preferably close to 1.2.

4. Process according to one of Claims 1 to 3, in which the temperature is allowed to rise during the introduction of benzyl chloride so as to reach 80°C at the end of introduction.

**Patentansprüche**

1. Verfahren zur Herstellung von Benzylmercaptan durch Umsetzung von Benzylchlorid mit Ammoniumhydrogensulfid in einem Mol-Verhältnis von $NH_4SH/C_6H_5CH_2Cl$ von wenigstens 1, dadurch gekennzeichnet, daß man die Reaktion in einem geschlossenen Reaktor unter Reaktionsdruck in zwei Stufen durchführt, wobei die erste im Einleiten des Benzylchlorids in eine wäßrige Ammoniumhydrogensulfidlösung bei einer Temperatur unter 80°C und die zweite im Erhitzen des Reaktionsgemischs auf eine Temperatur von 80 bis 100°C besteht.

2. Verfahren nach Anspruch 1, dadurch gekennzeichnet, daß die Konzentration der wäßrigen Ammoniumhydrogensulfidlösung zwischen 25 und 40 Gew.% beträgt.

3. Verfahren nach Anspruch 1 oder 2, dadurch gekennzeichnet, daß das Molverhältnis von $NH_4SH/C_6H_5CH_2Cl$ zwischen etwa 1,05 und 1,5 und vorzugsweise etwa 1,2 beträgt.

4. Verfahren nach einem der Ansprüche 1 bis 3, dadurch gekennzeichnet, daß man während des Einleitens von Benzylchlorid die Temperatur derart steigen läßt, daß sie am Ende dieses Vorgangs 80°C erreicht.